# EUROPÄISCHE PATENTANMELDUNG

(11) **EP 2 141 512 A2**
(43) Veröffentlichungstag der Anmeldung: **06.01.2010**
(21) Anmeldenummer: 09158108.2
(22) Anmeldetag: 17.04.2009
(51) Int. Cl.: G01T 1/29

(54) **Verfahren zur Energieüberprüfung eines Partikelstrahls, Vorrichtung zur Energieüberprüfung sowie Anlage hiermit**

(30) Priorität: 27.06.2008 DE 102008030699
(71) Anmelder: Siemens Aktiengesellschaft, 80333 München (DE)
(72) Erfinder: Rietzel, Eike, 64289, Darmstadt (DE); Tober, Nils, 12437, Berlin (DE)

(57) **Zusammenfassung**

Verfahren zur Energieüberprüfung eines Partikelstrahls, Vorrichtung zur Energieüberprüfung sowie Anlage hiermit

Die Erfindung betrifft ein Verfahren zur Energieüberprüfung eines Partikelstrahls, aufweisend folgende Schritte:
- Beschleunigen von geladenen Partikeln auf eine vordefinierte Energie in einer Beschleunigungsvorrichtung (13,15),
- Extrahieren eines Partikelstrahls aus der Beschleunigungsvorrichtung und Führen des Partikelstrahls durch eine Transportvorrichtung (17),
- Umlenken des Partikelstrahls mit zumindest einem Magneten (31, 49),
- Messen einer Position des Partikelstrahls in einer Richtung, die idealerweise, aber nicht zwingend senkrecht zur Strahlrichtung verläuft,
- Überprüfen einer tatsächlichen Energie des Partikelstrahls unter Verwendung der gemessenen Position.

Die Vorrichtung zur Energieüberprüfung umfasst eine Messvorrichtung (37) zum Messen einer Position des Partikelstrahls in einer Richtung, die senkrecht zur Strahlrichtung verläuft, und eine Auswertungsvorrichtung (39) zum Überprüfen einer tatsächlichen Energie des Partikelstrahls unter Verwendung der von der Messvorrichtung (47) gemessenen Position. Eine derartige Vorrichtung findet in einer Anlage zum Beschleunigen von geladenen Partikeln Einsatz.

## Beschreibung

Die Erfindung betrifft ein Verfahren zur Energieüberprüfung eines Partikelstrahls. Weiterhin betrifft die Erfindung eine Vorrichtung zur Energieüberprüfung sowie eine Anlage mit einer derartigen Vorrichtung. Ein derartiges Verfahren bzw. eine derartige Vorrichtung finden insbesondere im Rahmen der Partikeltherapie Einsatz, bei der beispielsweise bei routinemäßig wiederholt durchzuführenden Qualitätssicherungs-Maßnahmen die Energie eines Partikelstrahls überprüft wird.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, finden jedoch auch in nicht-therapeutischen Gebieten Anwendung. Hierzu gehören beispielsweise Forschungsarbeiten im Rahmen der Partikeltherapie, die an nicht-lebenden Phantomen oder Körpern durchgeführt werden, Bestrahlungen von Materialien, etc. Hierbei werden geladene Partikel wie z.B. Protonen oder Kohlenstoffionen oder andere Ionensorten auf hohe Energien beschleunigt, zu einem Partikelstrahl geformt und über ein Hochenergiestrahltransportsystem zu einem oder mehreren Bestrahlungsräumen geführt. In einem dieser Bestrahlungsräume wird das zu bestrahlende Objekt mit dem Partikelstrahl bestrahlt.

Hierbei ist es für den Erfolg einer Bestrahlung wesentlich, dass die Anlage fehlerfrei betrieben werden kann. Üblicherweise wird dies dadurch gewährleistet, dass im Rahmen von regelmäßigen Qualitätssicherungs-Maßnahmen (auch QA-Maßnahmen genannt, QA für engl.: "quality assurance") das korrekte Funktionieren der Anlage überprüft wird. Insbesondere wird hierbei überprüft, ob ein Partikelstrahl, der mit bestimmten Spezifikationen angefordert wird, auch tatsächlich die geforderten Spezifikationen aufweist.

Eine Eigenschaft des Partikelstrahls, die korrekt eingestellt werden muss, ist die Energie des Partikelstrahls, da die Energie der Partikel die Eindringtiefe des Partikelstrahls in ein zu bestrahlendes Objekt bestimmt.

Zur Überprüfung der Energie des Partikelstrahls ist die Verwendung einer so genannten "Wassersäule" oder eines "Wasserphantoms" bekannt. Hierbei ist eine Ionisationskammer in dem Wasserphantom bzw. in der Wassersäule angeordnet, auf die der Partikelstrahl gerichtet wird. Diese Ionisationskammer wird in Strahlrichtung im Wasser bewegt und liefert unterschiedliche Messwerte. Das (Messwert-)Maximum in der Tiefe korreliert mit der Reichweite der Partikel. Dieses Verfahren wird unter anderem bei QA-Maßnahmen eingesetzt. Die Messungen eine Energie bzw. die Ermittlung eines Bragg-Peak dauert jedoch circa 3 bis 5 Minuten pro Energie, evtl. auch etwas weniger. Im Rahmen einer klinischen Konstanz Prüfung muss zudem ein großer Energiebereich geprüft werden. Zudem addieren sich die Aufbauzeiten für die Messvorrichtung.

Bisher nur bei passiven Strahlapplikation, das heißt bei Aufweitung des Partikelstrahls durch einen Streukörper, wird zum Teil auch ein so genannter "multi-layer Faraday cup" eingesetzt. Dabei wird durch mehrere, hintereinander positionierte Faraday-Auffänger ("Faraday-Becher") jeweils die Ionisation in unterschiedlichen Tiefen gemessen, um die Reichweite des aufgeweiteten Partikelstrahls grob zu bestimmen.

Es ist die Aufgabe der Erfindung, ein Verfahren zur Energieüberprüfung eines Partikelstrahls anzugeben, das ohne Aufbauten einer aufwändigen Messvorrichtung betrieben werden kann und das schnell durchführbar ist. Weiterhin ist es die Aufgabe der Erfindung, eine Vorrichtung zur Energieüberprüfung sowie eine Anlage zum Beschleunigen von geladenen Partikeln anzugeben, mit der bzw. bei der eine Überprüfung der Energie eines Partikelstrahls ohne aufwändige Aufbauten schnell durchführbar ist.

Die Aufgabe wird gelöst durch ein Verfahren gemäß Anspruch 1, durch eine Vorrichtung zur Energieüberprüfung gemäß Anspruch 9 sowie durch eine Anlage gemäß Anspruch 10. Vorteilhafte Weiterbildungen finden sich in den Merkmalen der abhängigen Ansprüche.

Das erfindungsgemäße Verfahren zur Energieüberprüfung eines Partikelstrahls in einer Partikeltherapieanlage weist folgende Schritte auf:
- Beschleunigen von geladenen Partikeln auf eine vordefinierte Energie in einer Beschleunigungsvorrichtung der Partikeltherapieanlage,
- Extrahieren eines Partikelstrahls aus der Beschleunigungsvorrichtung und Führen des Partikelstrahls zu einem Bestrahlungsraum der Partikeltherapieanlage durch eine Transportvorrichtung,
- Umlenken des Partikelstrahls mit zumindest einem Umlenkmagneten,
- Messen einer Position des Partikelstrahls in einer Richtung, die eine Komponente senkrecht zur Strahlrichtung aufweist,
- Überprüfen einer tatsächlichen Energie des Partikelstrahls unter Verwendung der gemessenen Position.

Das Verfahren beruht auf der Idee, dass eine Ablenkung des Partikelstrahls in einem Magnetfeld energieabhängig ist. Die Lorentzkraft, die eine Ablenkung eines geladenen Teilchens im Magnetfeld bewirkt, hängt von dem Magnetfeld und von der Geschwindigkeit des Teilchens ab. Ist die Energie des Partikelstrahls und das Magnetfeld bekannt, kann eine Sollposition des Partikelstrahls an einem beliebigen Messpunkt entlang der Strahlrichtung ermittelt werden. Weicht aber die gemessene Position des Partikelstrahls im Messpunkt von der Sollposition ab, so deutet dies darauf hin, dass die tatsächliche Energie des Partikelstrahls nicht der vordefinierten Energie entspricht. Die vordefinierte Energie entspricht üblicherweise einer Energie, mit einer z.B. eine Bestrahlung eines Zielobjekts, z.B. eines Patienten oder eines Phantoms zu Forschungs- oder Kalibrierungszwecken, erfolgen kann.

Die gemessene Position und die Sollposition unterscheiden sich, da der Partikelstrahl von einer Soll-Verlaufsrichtung abweicht. Diese Abweichung kann in einer Richtung gemessen werden, die nicht parallel zur Strahlverlaufsrichtung ist, die also eine Komponente aufweist, welche Komponente senkrecht zur Verlaufsrichtung angeordnet ist. Idealerweise ist diese Richtung im Wesentlichen senkrecht zur Strahlrichtung angeordnet, d.h. in einem Winkelbereich von 90° ± 15°, 90° ± 10°, 90° ± 5° oder noch weniger. Dies ist aber nicht zwingend notwendig. Es ist auch denkbar, diese Richtung in einem spitzen bzw. stumpfen Winkel zur Strahlverlaufsrichtung anzuordnen, der mehr als 15° von der Strahlrichtung abweicht. Je mehr der Winkel von der Senkrechten abweicht, desto aufwändiger wird es, eine Messvorrichtung so auszubilden, dass auch kleine Abweichungen des Strahlverlaufs von der Soll-Verlaufsrichtung detektiert werden können.

Insbesondere in einer Anlage, in der die Partikel beschleunigt werden und über die Transportvorrichtung in einen Bestrahlungsraum geführt werden, wie beispielsweise in einer Partikeltherapieanlage, kann das Verfahren zur Überprüfung der Energie des Partikelstrahls eingesetzt werden.

Insbesondere wird die tatsächliche Energie des Partikelstrahls auf eine Abweichung in Bezug auf die vordefinierte Energie des Partikelstrahls überprüft.

Verglichen mit Verfahren, bei denen die Überprüfung der Energie durch Ermittlung der Lage des Bragg-Peaks in einer Wassersäule oder einem Wasserphantom durchgeführt wird, ist das Verfahren wesentlich schneller und einfacher.

Das Überprüfen, ob der Partikelstrahl die gewünschte, vordefinierte Energie tatsächlich aufweist, kann z.B. dadurch erfolgen, dass aus der Position des Partikelstrahls die tatsächliche Energie des Partikelstrahls berechnet wird. Dies ist möglich, da aus der Position des Partikelstrahls auf die Geschwindigkeit der Partikel geschlossen werden kann und da die Magnetfeldstärke des Umlenkmagneten - oder die Magnetfeldstärken der Umlenkmagnete, wenn mehrere Umlenkmagnete verwendet werden - und der geometrische Verlauf des Partikelstrahls bekannt ist. Zur Berechnung werden bekannte physikalische Zusammenhänge wie die Lorentzkraft, der Energie-Impuls-Zusammenhang bei einem bewegten Teilchen, etc. verwendet. Die Magnetfeldstärke des Umlenkmagneten bzw. der Umlenkmagnete und deren korrekte Einstellung kann zusätzlich überprüft oder sichergestellt werden, z.B. über eine Magnetfeldmessung und/oder Feldstärkeregelung. Das Messen der Position erfolgt an einem Ort, der sich - in Strahlrichtung gesehen - nach dem Ort befindet, an dem der Partikelstrahl umgelenkt worden ist.

Die quantitative Bestimmung kann dabei die absolute Energie des Partikelstrahls oder die relative Energiedifferenz zu der voreingestellten Energie oder die relative Energiedifferenz zwischen zwei voreingestellten Energien ermitteln. Die Berechnung muss dabei nicht die exakte tatsächliche Energie des Partikelstrahls erlauben, es kann je nach gewünschter Genauigkeit ausreichen, lediglich eine näherungsweise Berechnung der Energie durchzuführen. Der Zusammenhang zwischen dem Ort des Partikelstrahls und der tatsächlichen Energie des Partikelstrahls kann auch in einer Rechnereinheit, beispielsweise in einer Tabelle, hinterlegt sein. Hierdurch ist eine explizite Berechnung anhand von einer Formel nicht notwendig.

Ebenso muss die Überprüfung nicht notwendigerweise mit einer quantitativen Bestimmung der Energie des Partikelstrahls verbunden sein. Es ist sogar denkbar, eine qualitative Überprüfung durchzuführen. Beispielsweise kann in letzterem Fall ein Signal erzeugt werden, wenn im Rahmen des Verfahrens die gemessene Position des Partikelstrahls von einer zu erwartenden Position zu sehr abweicht, wenn die Abweichung z.B. über einem Schwellenwert liegt. In diesem Falle ist durch die Positionsmessung festgestellt worden, dass die tatsächliche Energie des Partikelstrahls zu stark von der vordefinierten Energie abweicht. Dies kann z.B. Anlass dafür sein, die Anlage zu warten.

Ein derartiges Verfahren hat den Vorteil, dass es schnell und einfach implementiert und automatisiert werden kann. Es benötigt keinen komplizierten Aufbau eigens dafür vorgesehene Messvorrichtungen und kann insgesamt innerhalb weniger Sekunden durchgeführt werden.

Ein derartiges Verfahren kann auch mit anderen Verfahren kombiniert werden, die zur Überprüfung der Energie des Partikelstrahls eingesetzt werden. So kann z.B. ein aufwändigeres Verfahren, wie es im Stand der Technik bekannt ist und mit dem eine sehr genaue Messung der Energie möglich ist - z.B. Messung der Energie des Partikelstrahls mit einem Prüfkörper wie z.B. einem Wasserphantom - in bestimmten Wartungsintervallen durchgeführt werden. Zwischen diesen Wartungsintervallen kann das erfindungsgemäße Verfahren eingesetzt werden, um die Energie des Partikelstrahls häufiger zu überprüfen.

In einer einfachen Ausführungsform der Erfindung wird der Partikelstrahl isozentrisch ausgerichtet. Dies bedeutet, dass eventuelle Steuer- oder Scan-Magnete in der Transportvorrichtung derart eingestellt werden, dass der Partikelstrahl das Isozentrum in einem Bestrahlungsraum treffen würde, sofern die tatsächliche Energie des Partikelstrahls der gewünschten, vordefinierten Energie entspricht. In einer Partikeltherapieanlage ist diese Einstellung besonders einfach zu erreichen, da eine derartige Anlage üblicherweise so ausgelegt ist, dass der Partikelstrahl - ohne weitere Ablenkung durch Scan-Magnete - das Isozentrum treffen würde.

In einer Ausführungsvariante wird der Partikelstrahl derart gesteuert, dass der Partikelstrahl sukzessive in unterschiedlichen, voreingestellten Ausmaßen abgelenkt wird, wobei die Position des Partikelstrahls mehrfach gemessen wird, und wobei die tatsächliche Energie des Partikelstrahls unter Verwendung der gemessenen Positionen überprüft wird. Insbesondere kann die relative Lage der gemessenen Positionen zur Ermittelung der Energie des Partikelstrahls verwendet werden.

Mit dieser Ausführungsform kann das Verfahren besonders genau ausgestaltet werden. Indem ein Punktmuster durch den Partikelstrahl angefahren wird, werden mehrere Messpunkt der an verschiedenen Positionen gemessen. Die tatsächliche Lage und/oder Anordnung der Messpunkte gibt Aufschluss über die Energie des Strahls. Hierdurch kann z.B. die Energie des Strahls redundant bestimmt werden, wodurch sich die Sicherheit des Verfahrens erhöht. Speziell die relative Lage der Punkte zueinander lässt einen einfacheren Rückschluss auf die Energie des Strahls zu, da nun nicht mehr die absolute Position eines Messpunktes im Raum, die komplizierter zu ermitteln ist, notwendig ist.

Es kann beispielsweise ein regelmäßiges Muster mit definierten Abständen zwischen den Positionen bestrahlt werden, in einem einfachen Fall die Eckpunkte eines Quadrats. Wenn die Energie des Teilchenstrahls der erwarteten bzw. geplanten Energie entspricht, werden die Abstände zwischen den Punkten wie erwartet gemessen. Bei Abweichungen der gelieferten Teilchenenergie verkürzen bzw. verlängern sich die Abstände zwischen de n Eckpunkten entsprechend.

Wenn der Partikelstrahl sukzessive in unterschiedlichen Ausmaßen abgelenkt wird, kann auch besser darauf geschlossen werden, ob eine Abweichung auf eine fehlerhafte Energieeinstellung oder auf eine fehlerhafte Strahlführung zurückzuführen ist. Wenn der Partikelstrahl z.B. mithilfe eines Scan-Magneten zweimal abgelenkt wird, und zwar in entgegengesetzter Richtung zu einer Nulllage, ist bei einer fehlerhaften Energieeinstellung die Abweichung in beiden Richtungen von den Sollpositionen in etwa gleich groß. Die Abweichungen korrelieren also in etwa miteinander. Wenn die Abweichungen in beiden Richtungen von den Sollpositionen hingegen unterschiedlich sind, ist dies eher darauf zurückzuführen, dass z.B. bei einem Scan-Magneten Einstellungen nicht korrekt sind oder dass die Strahlführung nicht korrekt ist, sodass der Strahl nicht an erwarteter Stelle in den Umlenkmagneten eintritt. In diesem Fall korrelieren die Abweichungen nicht miteinander.

In einer Ausführungsvariante werden die Partikel in einer Anlage beschleunigt, in welcher der Partikelstrahl über die Transportvorrichtung in einen Bestrahlungsraum geführt wird und dort aus einem Austrittsfenster austritt. Zur Positionsmessung wird eine erste Messvorrichtung verwendet, die in Strahlrichtung nach dem Austrittsfenster angeordnet ist. Diese Messvorrichtung kann beispielsweise im Isozentrum positioniert werden.

In einer anderen alternativen und/oder zusätzlichen Ausführungsvariante wird zur Positionsmessung eine zweite Messvorrichtung verwendet, welche zwischen dem Umlenkmagneten und dem Austrittsfenster angeordnet ist. Eine derartige Messvorrichtung ist üblicherweise - z.B. bei Partikeltherapieanlagen - bereits vorhanden, um die Position des Partikelstrahls zu überprüfen. Die Messvorrichtung ist z.B. in der so genannten BAMS (für engl.: "beam application and monitoring system") am Ende der Transportvorrichtung angeordnet und dient im regulären Betrieb der Anlage dazu, die Position des Partikelstrahls bei der Durchführung einer Bestrahlung zu messen und zu kontrollieren. In diesem Fall ist keine zusätzliche Messvorrichtung zur Durchführung des Verfahrens notwendig, es wird die bereits vorhandene Messvorrichtung verwendet.

Als Messvorrichtung kann eine Draht- oder Streifenkammer wie z.B. eine Vieldraht-Proportionalkammer (MWPC für engl: "multi wire proportional chamber") eingesetzt werden, wie sie in bekannter Weise bei Partikeltherapieanlagen zur Ortsmessung eingesetzt werden.

Wenn die Anlage derart ausgebildet ist, dass sie zumindest zusätzlich ein Steuerelement zur Ablenkung des Partikelstrahls aufweist - beispielsweise einen Scan-Magneten - kann die zweite Messvorrichtung für einen Regelmechanismus zur Ortskorrektur des Partikelstrahls verwendet werden. Der Ort des Partikelstrahls wird hierbei gemessen, mit einer Sollposition verglichen und das Steuerelement entsprechend eingestellt, so dass die Sollposition auch bei einer Abweichung erreicht wird. In diesem Fall wird bei der Durchführung des Verfahrens die Anlage jedoch so betrieben, dass der Regelmechanismus zur Ortskorrektur ausgeschaltet ist. Dies ist notwendig, da die Abweichung der Position des Partikelstrahls von einer Sollposition gerade zur Überprüfung der Energie des Partikelstrahls verwendet werden und nicht automatisch korrigiert werden soll. Eine andere Ausführungsvariante wird weiter unten näher erläutert.

Die erfindungsgemäße Vorrichtung zur Energieüberprüfung eines Partikelstrahls, der auf eine vordefinierte Energie beschleunigt ist, durch eine Transportvorrichtung geführt ist und aus einer geraden Verlaufsrichtung umgelenkt ist, weist auf:
- eine Messvorrichtung zum Messen einer Position des Partikelstrahls in einer Richtung, die eine Komponente senkrecht zur Strahlrichtung aufweist,
- eine Auswertungsvorrichtung zum Überprüfen einer tatsächlichen Energie des Partikelstrahls unter Verwendung der von der Messvorrichtung gemessenen Position.

Die erfindungsgemäße Partikeltherapieanlage umfasst:
- eine Beschleunigungsvorrichtung zum Beschleunigen von geladenen Partikeln auf eine vordefinierte Energie,
- eine Transportvorrichtung zum Führen des beschleunigten Partikelstrahls zu einem Bestrahlungsraum durch eine Transportvorrichtung,
- einen Magneten zum Umlenken des Partikelstrahls, und
- eine Vorrichtung zur Energieüberprüfung mit einer Messvorrichtung zum Messen einer Position des Partikelstrahls in einer Richtung, die eine Komponente senkrecht zur Strahlrichtung aufweist, und mit einer Auswertungsvorrichtung zum Überprüfen einer tatsächlichen Energie des Partikelstrahls unter Verwendung der von der Messvorrichtung gemessenen Position.

Die Vorrichtung zur Energieüberprüfung des Partikelstrahls bzw. die Partikeltherapieanlage mit einer derartigen Vorrichtung können dabei so ausgebildet sein, dass die unterschiedlichen Ausgestaltungen des Verfahrens mit der Vorrichtung zur Energieüberprüfung bzw. mit der Partikeltherapieanlage durchgeführt werden können.

Ausführungsformen der Erfindung sowie Weiterbildungen gemäß den Merkmalen der abhängigen Ansprüche werden anhand der folgenden Zeichnung näher erläutert, ohne darauf beschränkt zu sein. Es zeigen:
- Fig. 1: einen schematischen Überblick über eine Partikeltherapieanlage,
- Fig. 2: eine Darstellung zur Illustration der zu Grunde liegenden Idee anhand eines isozentrisch umgelenkten Partikelstrahls,
- Fig. 3: eine Darstellung zur Illustration der zu Grunde liegenden Idee anhand eines Partikelstrahls, der mit Hilfe von Scan-Magneten mehrfach in unterschiedlicher Weise abgelenkt wird,
- Fig. 4: eine Darstellung einer Feed-back-Regelschleife zur Ortskorrektur des Partikelstrahls, die bei Ausführungsformen des erfindungsgemäßen Verfahrens ausgeschaltet ist,
- Fig. 5: eine schematische Darstellung der Verfahrensschritte.

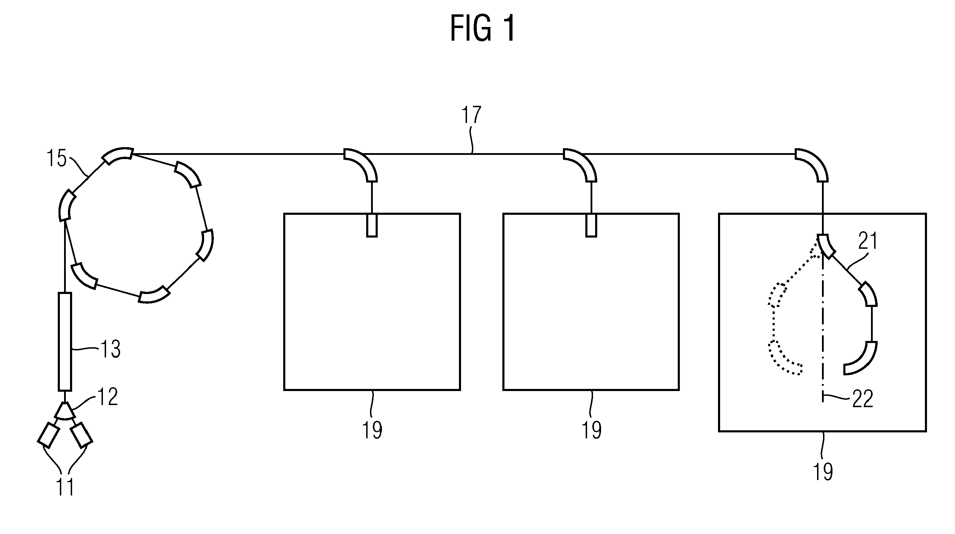

Figur 1 zeigt einen schematischen Überblick über den Aufbau einer Partikeltherapieanlage 10. In einer Partikeltherapieanlage 10 erfolgt insbesondere eine Bestrahlung eines Körpers, insbesondere eines tumorerkrankten Gewebes, mit einem Partikelstrahl.

Als Partikel werden vornehmlich Ionen wie beispielsweise Protonen, Pionen, Heliumionen, Kohlenstoffionen oder andere Ionensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Der anhand der Figur 1 dargestellte Grundaufbau einer Partikeltherapieanlage 10 ist beispielhaft für eine Partikeltherapieanlage, kann aber auch hiervon abweichen.

Die nachfolgend beschriebenen Ausführungsbeispiele sind sowohl im Zusammenhang mit der anhand von Fig. 1 dargestellten Partikeltherapieanlage als auch mit anderen Partikeltherapieanlagen oder allgemein in Anlagen anwendbar, in denen Partikel beschleunigt und in denen die Energie der beschleunigten Partikel überprüft werden soll.

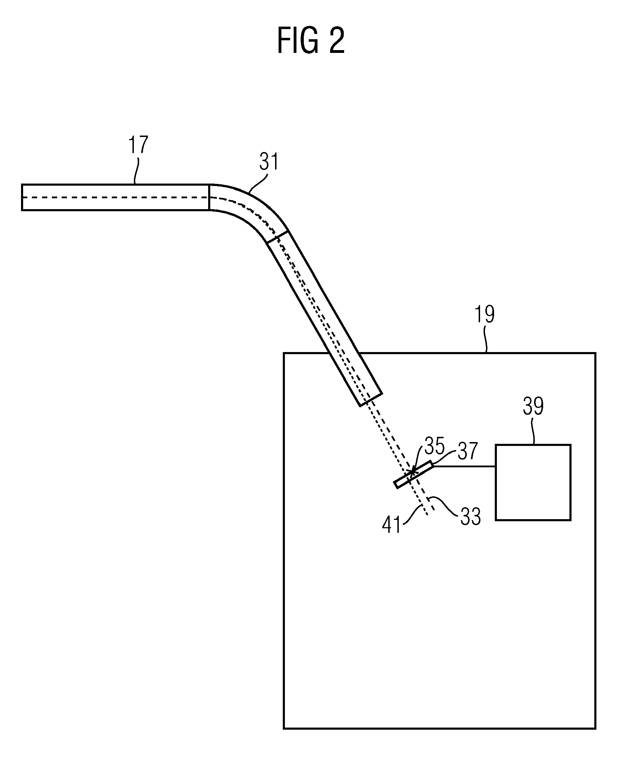

Fig. 2 zeigt ein Ausführungsbeispiel, bei dem der Partikelstrahl über das Hochenergiestrahl-Transportsystem 17 in einen Bestrahlungsraum 19 geführt wird und dabei von einem Umlenkmagneten 31 abgelenkt wird. Am Ende des Hochenergiestrahl-Transportsystems 17 tritt der Partikelstrahl aus einem Austrittsfenster 43 aus. Der Partikelstrahl ist dabei isozentrisch ausgerichtet, das heißt, dass die vordefinierte Energie des Partikelstrahls und die Magnetstärke des Umlenkmagneten 31 (und gegebenenfalls Einstellungen weitere Elemente im Hochenergiestrahl-Transportsystem 17) derart gewählt werden, dass der Partikelstrahl das Isozentrum 35 des Bestrahlungsraums 19 trifft - eine korrekte Einstellung der Betriebsparameter vorausgesetzt. Ein Partikelstrahl, bei dem alle Betriebsparameter korrekt eingestellt sind, bei dem also die tatsächliche Energie der vordefinierten, gewünschten Energie entspricht, ist mit einer gestrichelten Linie 33 dargestellt.

Am Isozentrum 35 ist ein Ortsdetektor 37 angeordnet, mit dem es möglich ist, die Position des Partikelstrahls in einer Richtung senkrecht zur Verlaufsrichtung des Partikelstrahls zu detektieren. Als Ortsdetektor 37 wird vorteilhafterweise eine Vieldraht-Proportionalkammer eingesetzt. Die Vieldraht-Proportionalkammer erlaubt die Erzeugung eines elektronischen Signals, das kennzeichnend für den Ort des Partikelstrahls ist und das auf einfache Weise in einer nachgeschalteten Rechnereinheit 39 ausgewertet werden kann. In der Rechnereinheit 39 erfolgt die Überprüfung der tatsächlichen Energie des Partikelstrahls.

Wenn beispielsweise der gemessene Ort des Partikelstrahls von dem Isozentrum 35 abweicht, kann daraus geschlossen werden, dass die tatsächliche Energie des Partikelstrahls nicht der vordefinierten Energie des Partikelstrahls entspricht. In Fig. 2 ist dies anhand des Partikelstrahls, der mit einer gepunkteten Linie 41 dargestellt ist, symbolisiert. Dieser Partikelstrahl wird durch den Umlenkmagneten 31 stärker abgelenkt und trifft daher nicht das Isozentrum 35. Durch Messung mit dem Ortsdetektor 37 kann nun festgestellt werden, dass dieser Partikelstrahl eine tatsächliche Energie hat, die geringer ist als die vordefinierte, gewünschte Energie.

Diese Überprüfung kann qualitativ oder aber auch quantitativ erfolgen, da die Abweichung des Partikelstrahls vom Isozentrum umso stärker ist, je mehr die tatsächliche Energie des Partikelstrahls von der vordefinierten Energie des Partikelstrahls abweicht.

Ein Umlenken des Partikelstrahls kann z.B. wie in Fig. 2 dargestellt durch einen Umlenkmagneten 31 erfolgen.

Ein Umlenken des Partikelstrahls kann aber auch z.B. durch so genannte Scan-Magnete erfolgen, mit denen der Partikelstrahl aus seiner Hauptachse abgelenkt wird, um so über ein Zielvolumen "gescannt" zu werden. Das durch Scan-Magnete erzeugte Magnetfeld ist üblicherweise kleiner als das Magnetfeld, das durch Umlenkmagnete erzeugt wird, die den Partikelstrahl in einen bestimmten Bestrahlungsraum leiten. Daher wird bei dem Verfahren zur Überprüfung der Energie der Ort des Partikelstrahls genauer gemessen werden, wenn das Umlenken des Partikelstrahls lediglich durch das vergleichsweise schwache Magnetfeld der Scan-Magnete erfolgt.

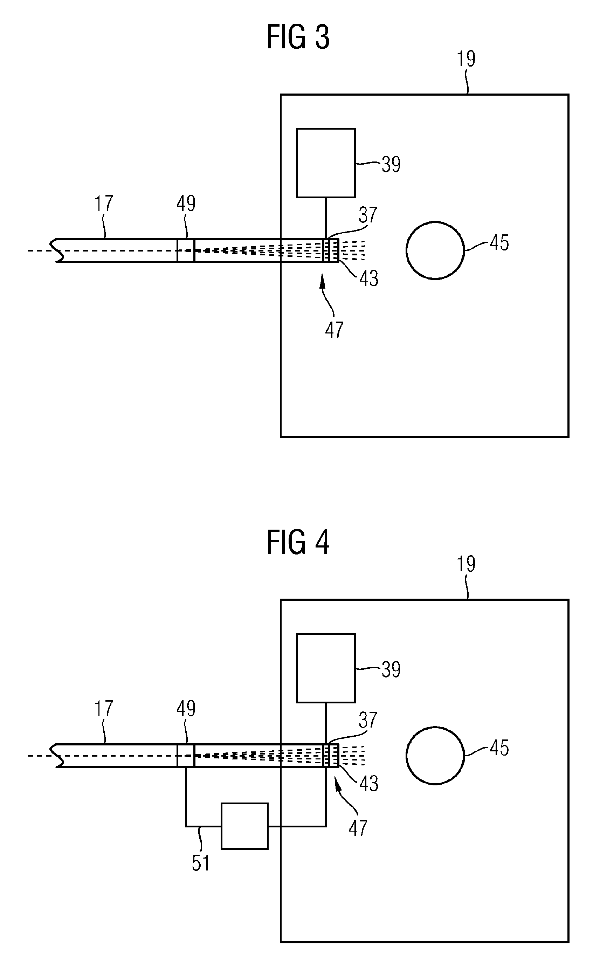

Fig. 3 zeigt ein derartiges Ausführungsbeispiel. Auch hier tritt der Partikelstrahl aus einem Austrittsfenster 43 aus dem Hochenergiestrahl-Transportsystem 17 aus, um nach kurzer Passage durch die Luft auf das zu bestrahlende Zielvolumen 45 zu treffen.

Vor dem Austrittsfenster 43 befindet sich üblicherweise eine Strahlapplikationsvorrichtung, auch BAMS 47 genannt (für engl.: "beam application and monitoring system"), mit der der Partikelstrahl kurz vor dem Austritt nochmals modifiziert werden kann und/oder mit der Parameter des Partikelstrahls kurz vor dem Austritt überprüft werden können. In einer BAMS 47 sind üblicherweise Ortsdetektoren wie Vieldraht-Proportionalkammern angeordnet, mit denen der Ort des Partikelstrahls in einer Ebene senkrecht zur Strahlverlaufsrichtung gemessen werden kann. Hier gezeigt ist ein Ortsdetektor 37 in der BAMS 47.

In Strahlrichtung vor der BAMS 47 befindet sich ein Scan-Magnet 49, mit dem die Verlaufsrichtung des Partikelstrahls während der Bestrahlung eines Zielvolumens 45 in einem gewissen Bereich verändert werden kann, so dass der Partikelstrahl über das Zielvolumen 45 z.B. gescannt wird. In einer Ausführungsform des Verfahrens wird nun der Scan-Magnet 49 derart angesteuert, dass der Partikelstrahl ein vordefiniertes Muster anfährt, bei dem der Partikelstrahl nacheinander an unterschiedliche Orte gelenkt wird. Die Position des Partikelstrahls wird dabei jeweils gemessen. Aus der relativen Lage der Orte zueinander kann nun auf die Energie des Partikelstrahls geschlossen werden.

Wenn der Abstand der einzelnen Positionen des Partikelstrahls zueinander größer ist, so ist die Energie des Partikelstrahls geringer, als bei einem Muster, bei dem der Abstand der einzelnen Positionen zueinander geringer ist - eine gleich bleibende Ansteuerung des Scan-Magneten 49 vorausgesetzt. Dies rührt daher, dass der Partikelstrahl durch das Magnetfeld des Scan-Magneten 49 bei geringerer Energie stärker abgelenkt wird und dadurch ein insgesamt größeres Muster erzeugt.

Anhand der relativen Lage der gemessenen Positionen zueinander kann die Energie des Partikelstrahls überprüft werden. Die Energie des Partikelstrahls kann dabei quantitativ oder auch lediglich qualitativ überprüft werden, beispielsweise durch einen Vergleich des tatsächlich gescannten Musters mit einem Soll-Muster. Wird eine zu große Abweichung festgestellt, kann ein Signal ausgegeben werden, das auf eine unzureichende Einstellung einer vordefinierten Energie des Partikelstrahls hinweist.

Der Übersichtlichkeit halber ist in Fig. 3 lediglich ein Ortsdetektor 37 und ein Scan-Magnet 49 gezeigt. Üblicherweise werden in einer Partikeltherapieanlage mehrere Scan-Magnete eingesetzt, mit denen der Partikelstrahl in unterschiedlichen Richtungen, beispielsweise in x-Richtung und in y-Richtung, abgelenkt werden kann. Ebenso werden üblicherweise mehrere Detektoren in einer Strahlapplikationsvorrichtung eingesetzt, um den Ort des Partikelstrahls in unterschiedlichen Richtungen und/oder redundant zu erfassen.

Fig. 4 zeigt ein Ausführungsbeispiel, das gegenüber dem Ausführungsbeispiel in Fig. 3 um eine Feed-back-Regelschleife 51 erweitert ist. Wenn der Partikelstrahl im regulären Betriebsmodus mithilfe der Feed-back-Regelschleife 51 über ein Zielvolumen 45 gescannt wird, werden kleine Abweichungen der tatsächlichen Position des Partikelstrahls von einer Sollposition ausgeglichen. Hierzu wird der Ort des Partikelstrahls nach Ablenkung durch den Scan-Magneten 49 mit dem Ortsdetektor 37 gemessen und der Istwert mit einem Sollwert verglichen. Die Feed-back-Regelschleife 51 steuert den Scan-Magnet 49 entsprechend an, um den Partikelstrahl an die gewünschte Sollposition zu führen.

Bei einer derartigen Ausgestaltung können der oder die Ortsdetektoren 37, die in die Feed-back-Regelschleife 51 eingebunden sind, auch zur Überprüfung der Energie des Partikelstrahls entsprechend dem erfindungsgemäßen Verfahren eingesetzt werden. Bei der Überprüfung der Energie des Partikelstrahls wird allerdings die Feed-back-Regelschleife 51 nicht verwendet. In diesem Fall wird nämlich die Abweichung des Ortes des Partikelstrahls gerade dazu verwendet, die Energie des Partikelstrahls zu überprüfen.

Alternativ könnte auch die Feed-back-Regelschleife bei der Bestrahlung verwendet werden und aus den in der Feed-back-Regelschleife ermittelten Korrekturdaten auf die Energie des Teilchenstrahls geschlossen werden. Damit wird dann nicht direkt der Ort als Maß für die Energie verwendet, sondern vielmehr aus der erforderliche Ortskorrektur des Partikelstrahls auf die korrekte Energieeinstellung geschlossen werden.

Die Ausführungsformen gemäß Fig. 1 bis Fig. 4 können auch miteinander kombiniert werden, beispielsweise kann ein Umlenken des Partikelstrahls sowohl an Umlenkmagneten erfolgen, mit denen der Partikelstrahl in einen speziellen Bestrahlungsraum gelenkt wird, als auch an Scan-Magneten, mit denen der Partikelstrahl über ein Zielvolumen gescannt wird. Ebenso ist es möglich, ein Umlenken des Partikelstrahls mithilfe der Strahlführung in einer Gantry 21 durchzuführen. Außerdem können auch Kombinationen der Positionsmessung, wie z.B. eine Positionsmessung in der BAMS und eine Positionsmessung am Isozentrum, eingesetzt werden.

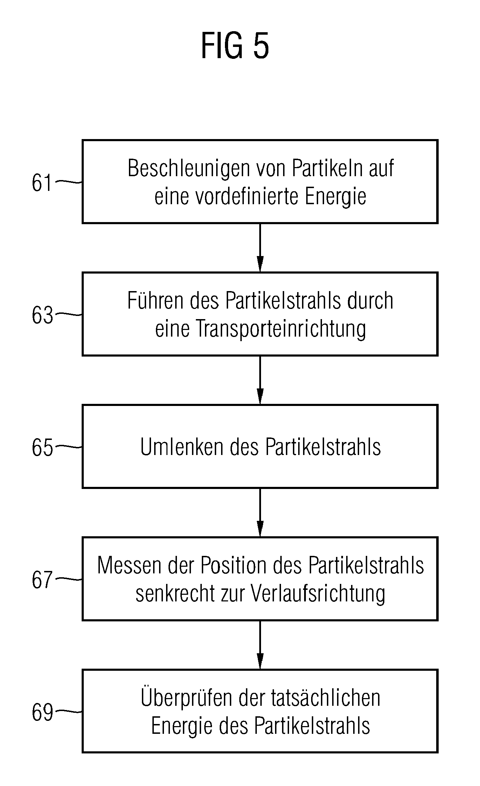

Fig. 5 zeigt eine schematische Darstellung einzelner Verfahrensschritte.

Anfangs werden die geladenen Partikel auf eine vordefinierte Energie beschleunigt (Schritt 61). Nach Beschleunigung der Partikel werden die Partikel entlang einer Transportvorrichtung geführt (Schritt 63) und mithilfe eines Magneten umgelenkt (Schritt 65). Nach Umlenken des Partikelstrahls erfolgt eine Messung der Position des Partikelstrahls in einer Richtung bzw. in einer Ebene, die senkrecht zur Verlaufsrichtung des Partikelstrahls verläuft (Schritt 67). Die gemessene Position des Partikelstrahls wird dazu verwendet, die tatsächliche Energie des Partikelstrahls insbesondere auf eine Abweichung von der vordefinierten Energie hin zu überprüfen (Schritt 69). Schritt 67 und Schritt 69 können insbesondere wiederholt ausgeführt werden, wobei bei jeder Wiederholung der Partikelstrahl in anderer Weise abgelenkt wird, so dass der Partikelstrahl auf andere Punkte im Raum gerichtet wird.

## Patentansprüche

1. Verfahren zur Energieüberprüfung eines Partikelstrahls in einer Partikeltherapieanlage (10), aufweisend folgende Schritte:
- Beschleunigen von geladenen Partikeln auf eine vordefinierte Energie in einer Beschleunigungsvorrichtung (13, 15) der Partikeltherapieanlage,
- Extrahieren eines Partikelstrahls aus der Beschleunigungsvorrichtung und Führen des Partikelstrahls durch eine Transportvorrichtung (17) zu einem Bestrahlungsraum (19) der Partikeltherapieanlage (10),
- Umlenken des Partikelstrahls mit zumindest einem Magneten (31, 49),
- Messen einer Position des Partikelstrahls in einer Richtung, die eine Komponente senkrecht zur Strahlrichtung aufweist,
- Überprüfen einer tatsächlichen Energie des Partikelstrahls unter Verwendung der gemessenen Position.

2. Verfahren nach Anspruch 1,
wobei die tatsächliche Energie des Partikelstrahls hinsichtlich einer Abweichung von der vordefinierten Energie des Partikelstrahls überprüft wird.

3. Verfahren nach einem der Ansprüche 1 bis 2, wobei der Bestrahlungsraum ein Isozentrum (35) aufweist und der Partikelstrahl isozentrisch ausgerichtet ist.

4. Verfahren nach einem der Ansprüche 1 bis 3,
- wobei der Partikelstrahl derart gesteuert wird, dass der Partikelstrahl sukzessive in unterschiedlichen Maßen abgelenkt wird,
- wobei bei jeder Ablenkung die Position des Partikelstrahls gemessen wird, und
- wobei die tatsächliche Energie des Partikelstrahls unter Verwendung der gemessenen Positionen überprüft wird, insbesondere unter Verwendung einer relativen Lage der gemessenen Positionen.

5. Verfahren nach einem der Ansprüche 1 bis 4, wobei zur Positionsmessung eine Messvorrichtung (37) verwendet wird, insbesondere eine Drahtkammer oder eine Streifenkammer.

6. Verfahren nach Anspruch 5,
wobei die Partikel in der Partikeltherapieanlage (10) beschleunigt werden, in welcher der Partikelstrahl über die Transportvorrichtung (17) in den Bestrahlungsraum (19) geführt wird und aus einem Austrittsfenster (43) austritt, und
wobei die Messvorrichtung (37) in Strahlrichtung nach dem Austrittsfenster (43) angeordnet ist.

7. Verfahren nach Anspruch 5,
wobei die Partikel in der Partikeltherapieanlage (10) beschleunigt werden, in welcher der Partikelstrahl über die Transportvorrichtung (17) in den Bestrahlungsraum (19) geführt wird und dort aus einem Austrittsfenster (43) austritt, und
wobei die Messvorrichtung (37) zwischen dem Magneten (31, 49) und dem Austrittsfenster (43) angeordnet ist.

8. Verfahren nach Anspruch 6 oder 7,
wobei die Messvorrichtung (37) in einem Regelmechanismus (51) zur Ortskorrektur des Partikelstrahls eingebunden ist, und
wobei die Anlage bei der Durchführung des Verfahrens derart betrieben wird, dass der Regelmechanismus (51) zur Ortskorrektur ausgeschaltet ist.

9. Verfahren nach Anspruch 6 oder 7,
wobei die Messvorrichtung (37) in einem Regelmechanismus (51) zur Ortskorrektur des Partikelstrahls eingebunden ist, und
wobei die Anlage bei der Durchführung des Verfahrens derart betrieben wird, dass Korrekturdaten für die Ortskorrektur, die in dem Regelmechanismus ermittelt werden, zur Energieüberprüfung verwendet werden.

10. Vorrichtung zur Energieüberprüfung eines Partikelstrahls, der auf eine vordefinierte Energie beschleunigt ist, durch eine Transportvorrichtung (17) geführt ist und aus einer geraden Verlaufsrichtung umgelenkt ist, die Vorrichtung aufweisend:
- eine Messvorrichtung (37) zum Messen einer Position des Partikelstrahls in einer Richtung, die eine Komponente senkrecht zur Strahlrichtung aufweist,
- eine Auswertungsvorrichtung (39) zum Überprüfen einer tatsächlichen Energie des Partikelstrahls unter Verwendung der von der Messvorrichtung (47) gemessenen Position.

11. Anlage zum Beschleunigen von geladenen Partikeln, aufweisend:
- eine Beschleunigungsvorrichtung (13, 15) zum Beschleunigen von geladenen Partikeln auf eine vordefinierte Energie,
- eine Transportvorrichtung (17) zum Führen des beschleunigten Partikelstrahls zu einem Bestrahlungsraum (19),
- einen Magneten (31, 49) zum Umlenken des Partikelstrahls, und
- eine Vorrichtung zur Energieüberprüfung mit einer Messvorrichtung (37) zum Messen einer Position des Partikelstrahls in einer Richtung, die eine Komponente senkrecht zur Strahlrichtung aufweist, und mit einer Auswertungsvorrichtung (39) zum Überprüfen einer tatsächlichen Energie des Partikelstrahls unter Verwendung der von der Messvorrichtung (37) gemessenen Position.

12. Anlage nach Anspruch 11,
die zur Durchführung eines Verfahrens nach einem der Ansprüche 2 bis 9 ausgebildet ist.
